# EUROPEAN PATENT SPECIFICATION

(11) **EP 4 609 795 B1**
(45) Date of publication and mention of the grant of the patent: **07.10.2026**
(21) Application number: 25159611.0
(22) Date of filing: 24.02.2025
(51) Int. Cl.: A61B 6/03, A61B 6/04, A61B 6/00

(54) **DEVICE FOR USE IN DYNAMIC RADIOLOGICAL IMAGE ACQUISITION**
VORRICHTUNG ZUR VERWENDUNG BEI DER DYNAMISCHEN ERFASSUNG RADIOLOGISCHER BILDER
DISPOSITIF DESTINÉ À ÊTRE UTILISÉ DANS L'ACQUISITION D'IMAGE RADIOLOGIQUE DYNAMIQUE

(30) Priority: 01.03.2024 EP 24160758
(43) Date of publication of application: 03.09.2025
(73) Proprietor: Vrije Universiteit Brussel, 1050 Brussel (BE)
(72) Inventor: BUZZATTI, Luca, Cambridge, CB4 2SP (GB); KEELSON, Benyameen, 1081 Koekelberg (BE); CATTRYSSE, Erik, 2990 Wuustwezel (BE); BULS, Nico, 1730 Asse (BE); VAN GOMPEL, Gert, 2800 Mechelen (BE); SCHEERLINCK, Thierry, 1390 Grez-Doiceau (BE); VANDEMEULEBROUCKE, Jef, 1083 Ganshoren (BE); MORENO BALLEN, Felipe, 1040 Brussels (BE); VAN DE VELDE, Gabriël-Mathieu Henri, 2870 Liezele (BE); DE MEY, Johan, 1700 Dilbeek (BE); VERSTRATEN, Tom, 1050 Elsene (BE)
(74) Representative: Winger

(56) References cited:
- US-A1- 2017 042 465
- US-A1- 2017 360 327
- US-A1- 2023 117 250

## Description

### Field of the invention

The present invention is generally related to the field of devices that can be used in medical care. More in particular, it relates to devices for creating a weight-bearing condition in medical images like musculoskeletal medical images.

### Background of the invention

Four-dimensional computed tomography (4DCT), also referred to as dynamic CT, is a type of CT scanning which records multiple image data sets over time. It allows playback of the scan as a video, so that physiological processes can be observed and internal movement can be tracked. The name is derived from the addition of time (as the fourth dimension) to traditional 3D computed tomography.

Dynamic CT is an upcoming technique to examine intra-articular and extra-articular pathologies as it provides excellent spatial and temporal resolution of bone motion. One of its main limitations with respect to lower limb acquisition is the fact that it is performed with the patient in a horizontal position and therefore in a non-weight bearing setting.

An example of such a set-up with the patient in horizontal position is found in the paper *"*Ability of novel foot and ankle loading device to reproduce loading conditions in the standing position during Computed Tomography" (T. Kimura et al., Journal of Medical Devices, Vol.9, Issue 4, 2015, 4 pages). The proposed loading device comprises a foot plate, a backboard and shoulder pads mounted on the backboard so that the shoulders support the load applied to the foot soles. An L-shaped foot plate is employed to apply an axial load to the soles in the cephalic direction. A rope capable of withstanding loads up to 1000 kg is threaded through holes on the foot plate, then through a pulley mounted to the backboard in the cephalic direction and left to hang towards the side of the platform of the CT scanner. Weight plates are hung from a hook attached to the end of the rope to reproduce loading conditions. The loading device of Kimura, however, does not allow movement of the legs : it only provides weight-bearing. So, it acquires images in a static position and does not allow acquiring real time dynamic CT data of the moving anatomy.

Also in the paper *"*An in vivo study of hindfoot 3D kinetics in stage II posterior tibial tendon dysfunction (PTTD) flatfoot based on weight-bearing CT scan" (Y.Zhang et al., Bone & Joint Research, vol.2, no.12, Dec.2013, pp.255-263) a custom made foot-loading device is presented to simulate normal full-body-weight-bearing using a counterbalance. The device comprises two main components: a foot plate with stress sensor and a main frame including a loading control component. A subject is positioned on the scanner bed in a supine position, with the right lower leg fixed to the supporting platform to keep it in a neutral position. The right foot sole is placed on a customised foot plate made of radiolucent materials. The foot plate is L-shaped and slides on the front edges of the main frame base. An external load (such as standard weights or sandbags) is applied to the foot plate through a system of wires and pulleys to simulate physical full-body-weight-bearing with the lower limb muscles under load. Again, this loading device does not allow movement of the legs. In addition, the device contains ferromagnetic materials which prevents its use in any imaging technique which uses magnetic properties, such as Magnetic Resonance Imaging (MRI).

There are cone-beam CTs that allow scanning the patient in orthostatic position (standing), see e.g. *"*Upright CT of the knee: the effect of weight-bearing on joint alignment" (Hirschmann et al., European Radiology (2015) 25:3398-3404). Such a standing CT scanner, however, cannot be used either, as it does not allow acquiring real time joint motion. Rather, it acquires static frames of the knee/ankle/etc in various discrete positions.

Some studies have investigated some aspect of the kinematics of the lower limb using four-dimensional computed tomography. For example, a study on the knee is presented in the papers *"*The relationship between tibial tuberosity-trochlear groove distance and abnormal patellar tracking in patients with unilateral patellar instability" (E.Williams et al., Arthrosc - J Arthrosc Relat Surg, 2016;32(1):55-61, available from: http://dx.doi.org/10.1016/j.arthro.2015.06.037) and *"*Correlation between Changes in Tibial Tuberosity-Trochlear Groove Distance and Patellar Position during Active Knee Extension on Dynamic Kinematic Computed Tomographic Imaging" (Tanaka et al., Arthrosc-J Arthrosc Relat Surg,. 2015;31(9):1748-55, from : http://dx.doi.org/10.1016/j.arthro.2015.03.015). However, although these studies apply 4DCT, they perform the movement in a non-weight-bearing condition of the lower limb.

When the lower limb is put under load during the physio(patho)logical movement, this may provide a better insight in the patient's pathology. A device to provide load during a dynamic movement of the lower limb was presented in WO2021/175827 A1. The device comprises a base structure and a table slidably mounted with respect to each other. The base structure is at one end provided with a platform allowing a patient to put his/her feet on. The device further comprises a counteracting structure that can apply to the table a load exerting force in a direction towards the end of the base structure provided with the platform from the opposite end of the base structure or to apply to the base structure a load exerting force in a direction from the one end provided with the platform to the opposite end of the base structure.

Although satisfactory in many ways, the solution proposed in WO2021/175827 suffers from an important drawback. When acquiring the radiological images, it is obviously required that the body part of interest, typically the knee, remain within the working field of the imaging modality, e.g. the CT, MRI or X-ray imaging device. It was found that with the set-up as described in the application this is not the case. Due to the movement of the body on the table the body part of interest moves in and out of the working field of the imaging device.

Hence, there is a need for an alternative implementation of the device wherein this limitation is dealt with.

### Summary of the invention

It is an object of embodiments of the present invention to provide for a device that can create loading conditions on a body part of interest of a person when acquiring radiological images, whereby during the acquisition at least a part of said body part of interest is kept within a predefined zone.

The above objective is accomplished by the solution according to the present invention.

In a first aspect the invention relates to a device for use with a weight-bearing radiological imaging modality scanner. The device comprises a base structure slidably mounted with respect to a table. The base structure is at one end provided with a platform making an angle relative to the table. The device further comprises a counteracting structure arranged to apply to the table a first load exerting force in a direction towards the one end provided with the platform from the opposite end of the base structure and to apply to the base structure a second load exerting force in a direction from the one end provided with the platform to the opposite end of the base structure.

The proposed solution indeed allows for creating a load condition on a body part of interest of the person being scanned, for example on the lower limb, whereby this part of interest can be kept within a predefined zone of the image field of the imaging modality scanner. The predefined zone may in some embodiments correspond to the full image field. In other embodiments the predefined zone may be a substantial part of the image field. The person lays down with the back on the table and the feet on the platform. First and second loads are applied that exert force to move on the one hand the table in the direction of the platform and on the other hand to move the base structure with the platform in a direction from the end of the base structure with the platform towards the opposite end of the base structure. Due to the simultaneously applied loads operating in opposite directions, the person bends the knees. The part of interest (typically, the ankle, the knees, the spine or the hip) thereby fully remains within the image field of the imaging modality being employed. Hence, the problem encountered with the device as proposed in the above-mentioned WO2021/175827 A1 is so overcome. This is achieved by appropriately choosing the values of the first load and the second load, thereby also taking into account one or more features that are specific to the person being scanned, for example the weight and/or one or more anthropometric measurements, without being limited thereto. The applied first load exerts a force towards the end of the base structure that lays closest to the person's feet. The person tries to resist to the applied force by pushing with both feet against the platform. On top of that, there is also a second load exerting force towards the opposite end of the base structure. The person takes a position as if he/she were squatting. The device is preferably used with a musculoskeletal four-dimensional radiological imaging modality. The, preferably dynamic, radiological images are acquired in real time while the person performs flexion-extension movements at the ankles, knees and hips. This allows studying real-time motion of internal structures and tissues, which may include, but are not limited to, bony structures, soft tissues (e.g. muscles, ligaments and tendons), vascular and nervous structures or organs. It is a major advantage of the device according to the invention that due to the first load and the second load operating in opposite directions, the body part of interest can be kept within the image field on the imaging modality scanner. In other embodiments the device can be used for obtaining static radiological images where the person performs no movement. In another embodiment, the static image can be obtained while a load is exerting a force (for instance in imaging of the spine, ankle, knee and/or hip) towards the end of the base structure that lays closest to the person's feet.

In preferred embodiments the base structure and the table are made in a radiolucent and/or non-ferromagnetic material. In this way image artefacts are avoided when acquiring the radiological images on any type of imaging modality. As such, it can be used with CT scanners, magnetic resonance imaging (MRI) scanners, positron emission tomography scanners in combination with CT (PET-CT), single photon emission computed tomography scanners in combination with CT (SPECT-CT), ultrasound imaging and any other type of X-ray imaging, e.g. 2D fluoroscopy imaging.

In embodiments the table is provided with adjustable shoulder supporting means. The shoulder supports protect the person's shoulders while pushing against the platform to resist the load.

In some embodiments of the invention the counteracting structure is movable.

In a preferred embodiment the counteracting structure is implemented with counterweights. The counteracting structure may then comprise a plurality of wheels that each are provided with a floor lock, so that a stable fixation to the floor can be achieved.

In another embodiment the counteracting structure is actuator-driven, e.g. motor-driven.

Advantageously, the device comprises stopping means at the end opposite the end provided with the platform for stopping movement of the table in a direction away from the platform.

In embodiments of the device according to the invention further stopping means are provided to keep the base structure in a fixed position with respect to the counteracting structure. In certain embodiments the further stopping means are a part of the counteracting structure.

In a preferred embodiment the device comprises a lock to keep the table in a fixed position with respect to the base structure. This increases safety when the person is positioned on the table.

In embodiments the device comprises a removable part to be put on the table in front of the platform, whereby the removable part is so dimensioned as to form an angle of substantially 90° relative to the table when combined with the platform.

In some embodiments the angle relative to the table is adjustable.

In another aspect the invention relates to a method for acquiring a weight-bearing radiological image of a part of interest of a person, using a device as previously described. The method comprises :
- mounting the device's table and base structure on a scanner table of a radiological imaging device,
- positioning a person with the feet on the platform of the base structure and with the part of interest inside an image field of view of a radiological imaging modality scanner of the radiological imaging device,
- applying a first load to the table of the device, said first load being related to one or more features specific to said person and exerting force in a direction towards an end of said base structure provided with the platform from the opposite end of the base structure, and applying a second load to the base structure of the device, said second load exerting force in a direction from the one end provided with the platform towards the opposite end of the base structure, said first and said second load configured to keep the part of interest within the image field,
- acquiring the weight-bearing radiological image of the body part of interest, while the person performs a movement to counter the force exerted by the first and the second load.

In a preferred embodiment the movement is a cyclic movement of flexion and extension of the person's ankle, knees and hips. The cyclic movement can be started and ended in any position between full extension and flexion.

In an embodiment the method comprises securing the table mounted on the base structure to the scanner table.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

### Brief description of the drawings

The invention will now be described further, by way of example, with reference to the accompanying drawings, wherein like reference numerals refer to like elements in the various figures.
Fig.1 illustrates an embodiment of the table and base structure with platform of the device according to the present invention.
Fig.2 illustrates an embodiment of the device wherein the base structure has lateral grooves and the table is provided with wheels.
Fig.3 illustrates the use of a removable part to create a 90° angle with the surface of the base structure.
Fig.4 illustrates an implementation of a counter-weight structure.
Fig.5 illustrates an embodiment wherein the mobile components are each attached to a different cam prescribing a specific motion.
Fig.6 illustrates an implementation of a motor-driven counter-weight structure.
Fig.7 illustrates the use of the device according to the invention.
Fig.8 illustrates another possible implementation of the counteracting structure.
Fig.9 illustrates SEM (mean and 95% CI) of right and left leg combined for the knee joint.

### Detailed description of illustrative embodiments

The present invention will be described with respect to particular embodiments and with reference to certain drawings but the invention is not limited thereto but only by the claims.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

It should be noted that the use of particular terminology when describing certain features or aspects of the invention should not be taken to imply that the terminology is being redefined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

Dynamic computed tomography (CT), also referred to as four-dimensional CT (4DCT), is a relatively new domain. Only recently it has been considered for studying the musculoskeletal system.

The present invention proposes in one aspect a device which allows examining a person's body part, for example a lower limb or a part thereof, during image acquisition by a real-time CT scan in a dynamic or static weight-bearing condition. If in dynamic weight-bearing condition, the device offers the possibility to properly load the leg during dynamic movement while performing a real-time dynamic CT scan of at least a part of interest of the person's body, for example the ankle, the knee or the hip. In this way new options for investigating, e.g., a person's lower limb, or at least said part of interest thereof, are created. Use of the proposed device allows for close kinetic chain movement. The segment more distant from the body, in this case the foot, is placed on a platform. The more proximal segments are allowed to move under a first load, while the segment fixed to the platform is allowed to move simultaneously under a second load. The segments move towards a predefined zone corresponding to the image field of the imaging modality or at least a substantial part thereof. The problem of the body part of interest moving in and out of the working field of the imaging device due to the body moving on the table, which was encountered when using the device presented in WO2021/175827 A1 is avoided. Movement of any joint in the kinetic chain causes a kinematic adjustment of the other joints. From a strict engineering perspective, the chain is "closed" if both ends are fixed to a common object, much like a closed circuit. Close kinetic chain movement is the closest condition of a physiological day-to-day activity movement like a squat.

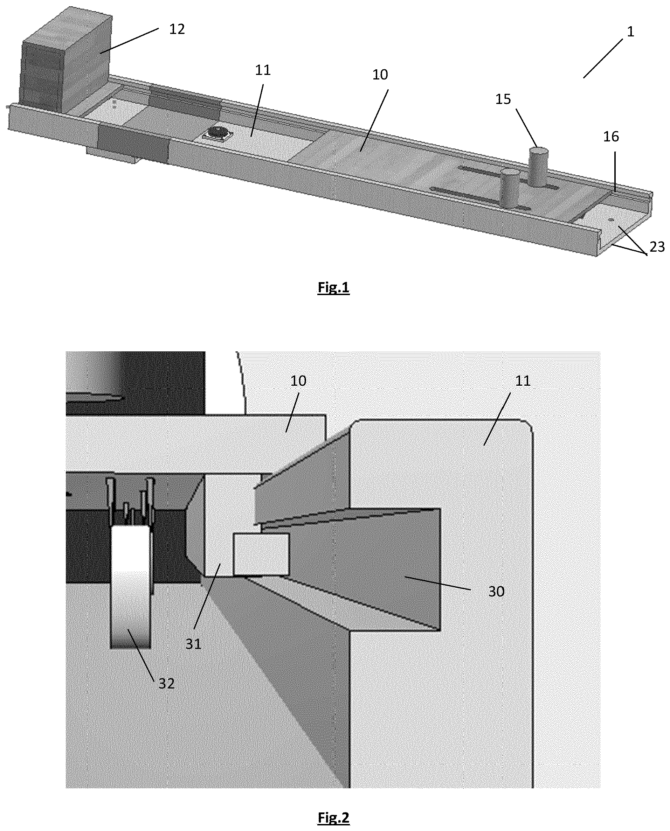

An illustration of an embodiment of the device according to the invention is provided in Fig.1. The device (1) comprises a table (10), also referred to as bed in this description, mounted on a fixed base structure (11). Depending on the imaging modality to be used, a predefined zone (13) can be indicated on the base structure that corresponds to the image field. The table can slide over the fixed base structure. The base structure is at one end provided with a platform (12) making an angle relative to the table. In the example of Fig.1 the angle is substantially 90°. Also this platform can slide over the base structure. The device (1) further comprises a counteracting structure (not shown in Fig.1) adapted to apply a load to the table and a load to the platform. Further details hereon are provided later in this description.

The device is intended to be put on top of a scanner table. When implementing the device, only radiolucent and/or non-ferromagnetic material is used for components, so that the artefacts in the acquired medical images can be avoided. In preferred embodiments the device is nearly entirely made of wood. The various wooden parts may be glued together in certain embodiments. In certain embodiments some parts that remain outside the field of view may be screwed together for a better fixation. In parts of the device that never enter the image field of view, non-radiolucent materials can be used. Further, the table and the base structure of the device have dimensions that match with the shape, e.g. concave shape, of the table. The base structure with the table on it is designed to avoid any physical contact with the sides of the scanner device.

In preferred embodiments the fixed base structure comprises two lateral rails or grooves (30) which can receive a fitting extension (31) of the bed. Reference is made to Fig.2. Wheels (32) are provided underneath the bed to allow movement over the bottom of the base structure. As such, the bed can slide along the base structure in a single longitudinal direction. Similarly, wheels are provided underneath the platform to allow movement.

The bed is in preferred embodiments covered with a cover pillow, e.g. made of foam. The cover itself may be in a washable material. The cover pillow may be removable.

Advantageously securing means are provided to keep the person in position on the table while the device is in use. In a preferred embodiment, as shown in Fig.1, the securing means are shoulder supports (15) on the upper side of the bed. As such, the person can exert a pushing force with the feet on the platform to resist the first load without sliding on the bed. This pushing force is to be added to the force in the same direction exerted by the second load. The shoulder supports may be adjustable in position, so that their position can be adapted to the size of the person lying on the bed. Further also the distance between the two supports can be adjusted. The shoulder supports are preferably made of a sanitary material that is washable. An alternative embodiment uses one or more belts, e.g. around the shoulders, instead of shoulder supports. The belts may be wound around the table (10).

In alternative embodiments the person can exert a pulling force on the platform instead of a pushing force. The platform can then be provided with a cable or a rope or a pair of cables or ropes to be held by the person being scanned to generate the pulling force. In such case the person's feet are preferably fixed to the platform. Shoulder supports on the upper side of the bed may be provided as well.

The base structure can be secured to the scanner table. The base structure comprises a number of fixation holes (23) which can be used for securing.

The sliding table can be locked on the base structure in order to allow a person to go on the table in a safe way. A lock (not shown in Fig.1) may be provided thereto in certain embodiments. At the end opposite the end provided with the platform the table may further be equipped with stopping blocks (16). The stopping blocks (16) limit the range (distance) over which the table can move in a direction away from said platform.

As already mentioned the base structure (11) is at one end provided with a movable platform (12) on which the person's feet can be placed. In some embodiments the platform makes a fixed angle with the flat surface of the base structure. In other embodiments the angle is adjustable. This adjusting may be performed manually or electrically, as is well known to the person skilled in the art. The angle can in some embodiments vary in a range between 100° and 140° or between 110° and 130°. Preferably the angle is about 120°. In other preferred embodiments the angle may be substantially 90°.

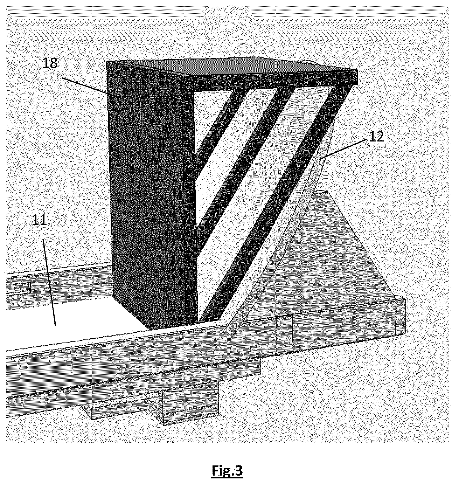

In an embodiment where the platform makes an angle of more than 90° relative to the table as illustrated in Fig.3, the device further comprises a removable part (18) to be put on the table in front of the platform. The removable part is movable along with the platform. The removable part is so dimensioned that when placed in front of the platform, a resulting angle of 90° relative to the table is obtained. Using this removable part allows investigating a situation as if the person were standing on the ground just as in case of a platform making an angle of substantially 90° as shown e.g. in Fig.1.

The device of the invention further comprises a counteracting structure to apply a load to the table.

In a preferred embodiment the counteracting structure is a counter-weight structure. The counter-weight structure is positioned at the same side with respect to the table as the platform. A counter-weight provides balance an stability to the device by applying an opposite force. The counter-weight structure is provided with at least two pulleys, i.e. at least one for the first load and at least one for the second load. A cable or a rope can be used to connect the weights. On the other side the cable or rope is attached to the distal end of the table, i.e. the end opposite the end with the platform. In certain embodiments the counter-weight structure may have a parallelepiped shape and be equipped with four wheels and a floor lock that allows lifting the entire structure.

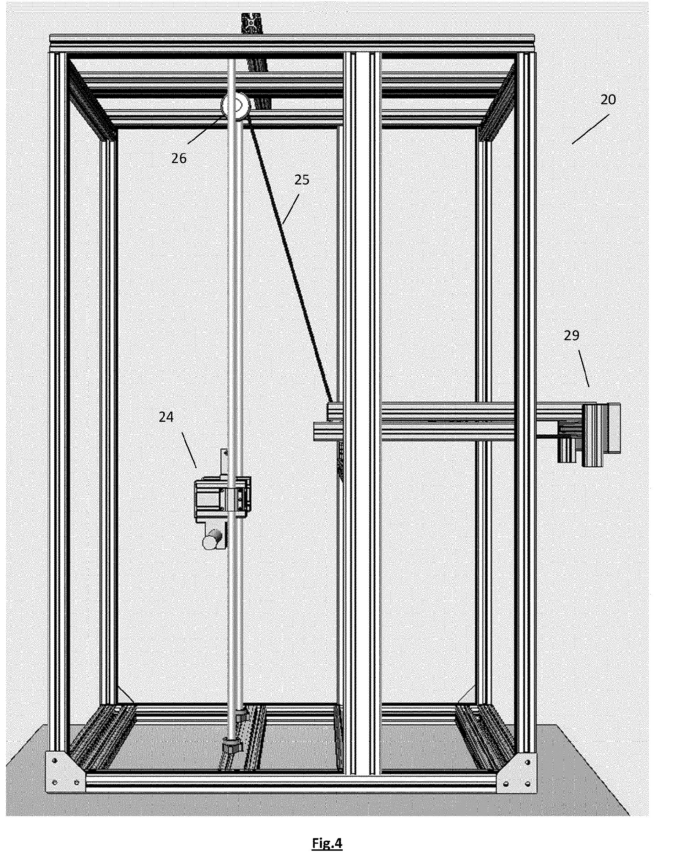

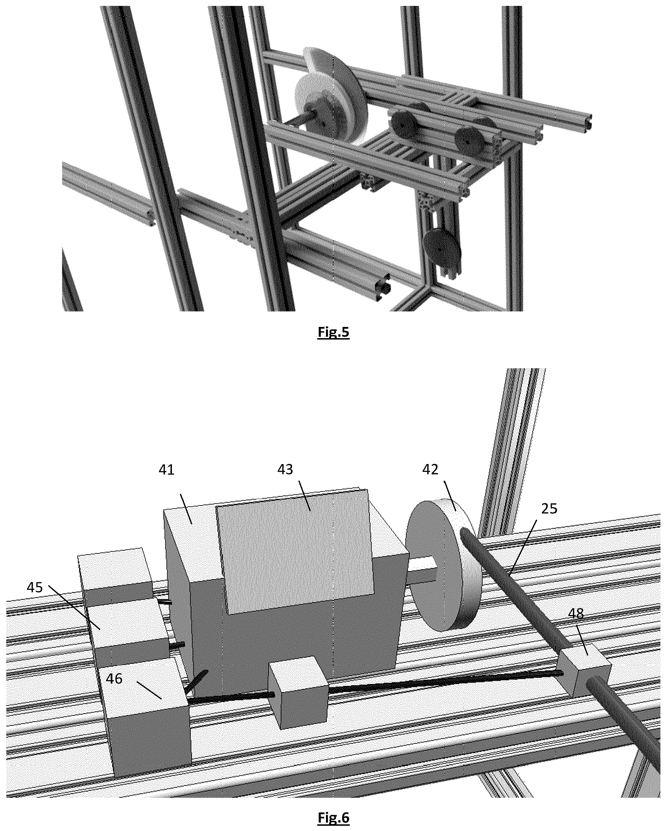

Fig.4 provides an illustration of a possible implementation of the counteracting structure (20) as a counter-weight system. The counter-weight system comprises at least two rotating and rounded components e.g. pulleys (26) or cams. The weights (24) are via a cable (25) connected to the table at its end opposite the end with the platform, as already explained previously. For reasons of clarity the platform is not shown in Fig.4. In the implementation of Fig.4 the counter-weight system is in direct contact with the floor. Fig.5 shows the two mobile components, platform and table, each attached to a different cam prescribing a specific motion.

In advantageous embodiments the device is equipped with stopping means (29) to keep the base structure in a fixed position with respect to the counteracting structure. In this way it is avoided that the base structure slide towards the counteracting structure, which might happen because the scanner table often cannot be maintained in a fixed position when the loads are applied. In an embodiment as in Fig.4 the stopping means is a part of the counteracting structure. In other embodiments the stopping means may be a part of the base structure.

In another preferred embodiment the counteracting structure is motor driven. The motor can then provide the required loads. The counteracting structure can be either mounted on a metal frame or attached at the end of the base structure (11). The latter option makes the entire system more compact as there is no need for the metal frame of the counteracting structure, but the former offers the advantage of a lighter and easier to manage base structure. The motor driven counteracting structure preferably incorporates a user-friendly display that allows setting the desired load. One option may be that information relating to the subject's weight and the body weight percentage or other person specific data like e.g. anthropometric measurement data is entered to obtain the corresponding first and second loads. The generated first and second loads always operate in the same respective direction. When the subject is pushing himself/herself away from the platform the motor generates a counteracting first load. The motor is connected to one of the cams shown in Fig.5 at a time. As a result of the two counteracting forces operating in opposite directions, the part of interest, e.g. typically the ankle, the knees, spine or hip, remains within the image field of the imaging modality. When the subject has reached full knees extension the motor generates a force to bring the table (10) and platform back to their original position. In other words, it acts in the same way as the weight that always provides a force in the direction of the counteracting structure. In a possible embodiment, see Fig.6, a cable (25) is fixed on a wheel (42) connected to a motor (41). The wheel revolves due to the action of the motor in the desired direction, thereby producing the desired torque and therefore desired load. The applied loads can be selected using a display (43), e.g. a touch screen. The counteracting structure can be implemented with one or more load cells (48) that allow providing feedback to the motor, through the controller unit (46), and adjusting the torque in real-time. The various functional blocks in Fig.6 receive power from a power supply (45).

In another embodiment the counteracting structure comprises for each of the required adjustable loads to be provided one or, preferably, more springs or one or, preferably, more rubber bands.

While in the above explanation a device for use in a musculoskeletal four-dimensional computed tomography image acquisition was presented, the invention relates in other aspects to a device suitable for use with any other radiological imaging modalities like e.g. magnetic resonance imaging (MRI), ultrasound, PET-CT, SPECT-CT, fluoroscopy and so on.

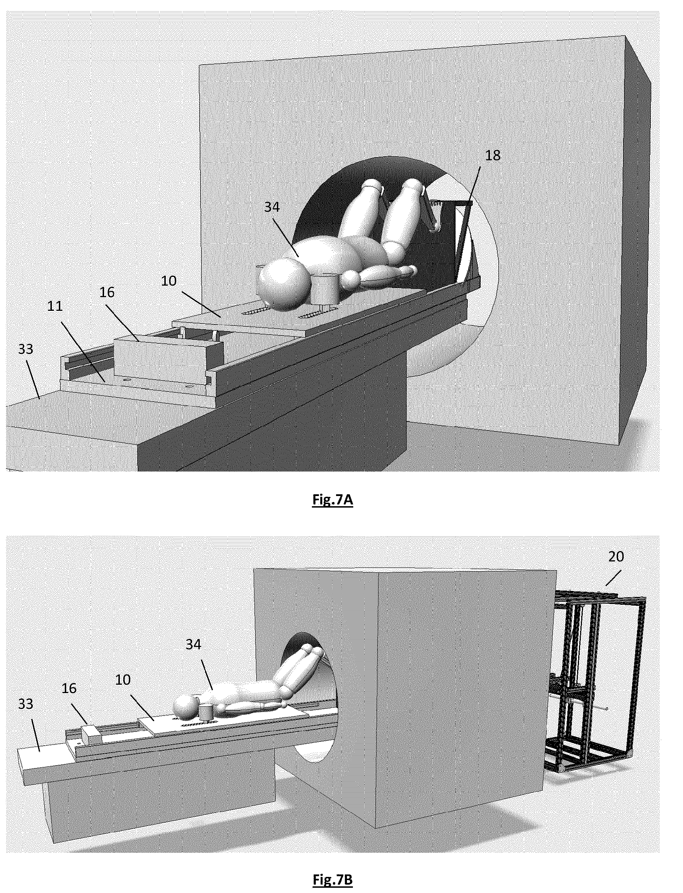

In another aspect the invention relates to a method for acquiring a musculoskeletal weight-bearing radiological image of a body part of interest of a person, wherein a device is used as described above. Typically, the body part belongs to the lower limb (e.g. the knees), but it may also be for example a hip. Figs.7A and 7B provide an illustration. The base structure with the table arranged thereon is mounted on and, preferably, secured to the scanner table (33). The person (34) is placed on the table with the feet on the platform of the base structure. The body part of interest of the person is positioned in a field of view of the scanner. Note that in Fig.7A the removable part (18) is used as well. A first load is applied to the table by means of the counteracting structure (e.g., with counterweights or motor-driven). The applied first load can be selected and may depend on one or more person-specific features like the person's weight and/or anthropometric measurement data (without being limited thereto) and on the goal of the study. The first load is such that the table is slid over the base structure in the direction of the platform, whereby the person's knees bend. The person exerts a counterforce in order to keep the table as much as possible in its original position by pushing with the feet against the platform. To make sure the body part of interest (e.g., of the lower limb) remains within a predefined zone corresponding to the image field of the imaging modality, or at least a substantial part thereof, a second load is applied to the base structure. This second load may in preferred embodiments also be related to one or more specific features like the person's weight, anthropometric measurement data etc... and exerts a force in a direction from the one end provided with the platform towards the opposite end of the base structure. The first and second load are configured so as to keep the body part of interest within the image field of the weight-bearing radiological imaging device. While doing so, the radiological images, e.g. 4DCT images, are acquired to record the movement in real time. The above-mentioned problem encountered when using the device presented in WO2021/175827 A1 is avoided.

As already mentioned, the device as previously described can readily be adapted to be mounted on an MRI machine. In this case, non-ferromagnetic material must be used for the table, the base structure and the counteracting structure. MRI can either be performed in a weight-bearing static position or during dynamic movement according to the clinical goal. The same procedure as in the previous paragraph is applied.

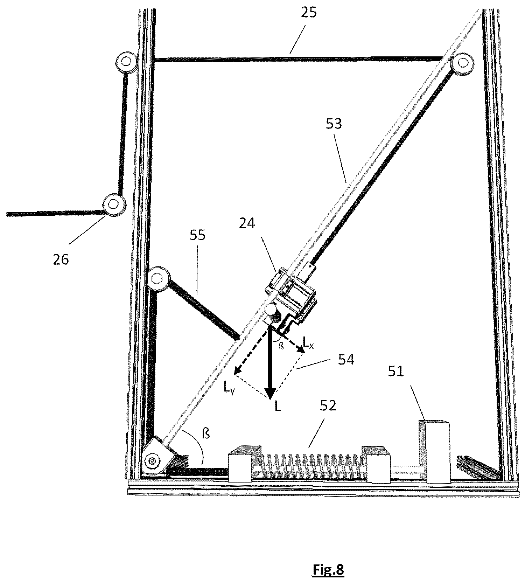

In another embodiment, the counteracting structure for at least one of the first and the second load can be implemented (see Fig.8) using a system of springs (52) that through the use of, among other things, one or more cables or belts (55) maintain one or more shafts (53) in equilibrium. Changing the position of the system of springs using an actuator (51) allows changing the angle β the shafts make with the metal structure. As the load L_{y} (i.e. the Y component of the load L) (54) transmitted through the cable (26) to the table depends on the angle β, the load can be adjusted by simply changing the angle β instead that changing the weights. The actuator control can be linked to, for example, a computer and operated via a graphical user interface.

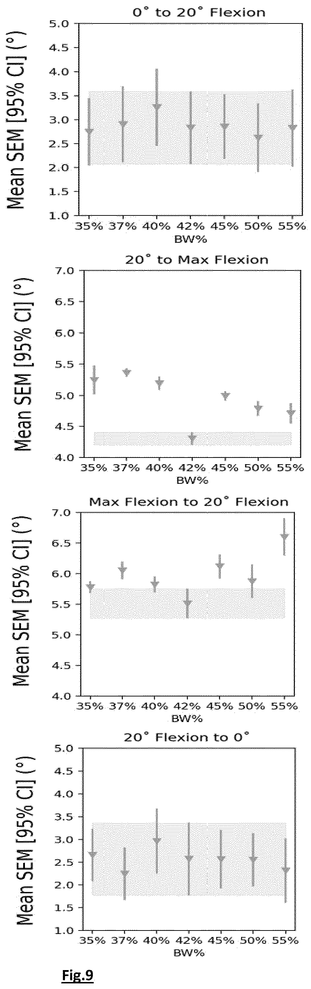

To validate the device of the invention tests are performed with a number of healthy subjects. The 3D-kinematics is captured with a camera system and muscle activation surface is recorded with surface electromyography (sEMG). The subjects are asked to perform two different tasks: a vertical squat against a door and a horizontal squat on the device. Subjects are allowed to familiarize for a few minutes with the two tasks after the subject knee flexion had been standardized. Reflective markers (pelvis, thigh, shank and foot) of the camera system and sEMG sensors (Rectus femoris (RF), Biceps femoris (BF), tibialis anterior (TA) and lateral Gastrocnemius (Ga) of both legs) are positioned. The subjects subsequently perform some repetitions of a vertical squat and of a horizontal squat for a number of sets. Every horizontal squat set has a number of different loading conditions, respectively 35%, 37%, 40%, 42%, 45%, 50% and 55% of the subject's bodyweight (BW%), that are randomized for each subject. To explore the degree of similarity of the left knee's 3D-kinematics, the two conditions are compared using Standard Error of Measurement (SEM) and median differences using the Euclidean Norm of the X,Y,Z axis. EMG activation is expressed as a percentage of maximum voluntary contraction (MVC) and similarity of the different horizontal squats are assessed using mean difference and Intraclass Correlation Coefficient (ICC). Mean SEM of the knee joint shows lowest values for 42% weight in the time interval between 20° of flexion and maximum flexion and between maximum flexion and 20° of the motion cycle, while no differences are seen in the start (0-20°) and end (20-0°) of the motion (Fig.9). A maximum difference between the two tasks across the entire motion is reported to not be higher than 10°. Mean ICC values of sEMG are shown to be highest for BW% 42, 45 and 37 (Table 1) resulting in an almost excellent reliability (ICC>0.8). Absolute differences between vertical and horizontal squat are smaller for BW% 55, 45 and 42 (Table 2) showing a difference of less than 9% of an MVC.

**Table 1 : ICC Mean difference between vertical and horizontal squat among subjects for sEMG. Right and left leg are combined**

| ICC | 35% | 37% | 40% | 42% | 45% | 50% | 55% |
|---|---|---|---|---|---|---|---|
| Rectus Femoris | 0.82 | 0.87 | 0.80 | 0.84 | 0.86 | 0.79 | 0.85 |
| Biceps Femoris | 0.65 | 0.65 | 0.58 | 0.63 | 0.72 | 0.74 | 0.66 |
| Tibialis Anterior | 0.65 | 0.74 | 0.65 | 0.79 | 0.63 | 0.55 | 0.70 |
| Gastrocnemius Lateralis | 0.82 | 0.87 | 0.93 | 0.94 | 0.93 | 0.86 | 0.67 |
| Mean | 0.74 | 0.78 | 0.74 | 0.80 | 0.79 | 0.73 | 0.72 |

**Table 2 : Absolute mean difference between vertical and horizontal squat among subjects. Right and left leg are combined**

| | 35% | 37% | 40% | 42% | 45% | 50% | 55% |
|---|---|---|---|---|---|---|---|
| ABS MEAN Rectus Femoris | 13,7 | 12,4 | 12,7 | 11,9 | 11,2 | 12,7 | 9,5 |
| SD | 8,8 | 8,1 | 9,0 | 9,1 | 8,4 | 9,0 | 8,3 |
| ABS MEAN Biceps Femoris | 3,2 | 2,6 | 3,2 | 2,9 | 2,9 | 3,2 | 2,5 |
| SD | 3,7 | 3,5 | 3,3 | 3,7 | 3,0 | 3,3 | 3,6 |
| ABS MEAN Tibialis Anterior | 19,2 | 18,3 | 17,3 | 15,0 | 16,0 | 17,3 | 14,0 |
| SD | 14,8 | 14,7 | 14,8 | 12,8 | 15,1 | 14,8 | 15,5 |
| ABS MEAN Gastrocnemius Lateralis | 7,1 | 6,1 | 4,6 | 5,0 | 4,8 | 4,6 | 7,0 |
| SD | 9,2 | 6,8 | 4,3 | 4,0 | 4,3 | 4,3 | 10,5 |
| ABS Mean TOT | 10,8 | 9,8 | 9,5 | 8,7 | 8,7 | 9,5 | 8,2 |
| SD TOT | 9,125 | 8,275 | 7,85 | 7,4 | 7,7 | 7,85 | 9,475 |

It can be concluded that the proposed device is able to reproduce lower limb kinematics when compared to an orthostatic wall squat.

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Device (1) for use with a weight-bearing radiological imaging modality scanner, the device comprising:
- a base structure (11) slidably mounted with respect to a table (10), said base structure being at one end provided with a platform (12) making an angle relative to said table, and
- a counteracting structure (20) arranged to apply to said table a first load exerting force in a direction towards said one end provided with said platform from the opposite end of said base structure;
**characterised in that** the counteracting structure is further arranged to apply to said base structure a second load exerting force in a direction from said one end provided with said platform to the opposite end of said base structure.

2. Device as in claim 1, wherein said base structure and said table are made in a radiolucent and/or non-ferromagnetic material.

3. Device as in claim 1 or 2, wherein said table is provided with adjustable shoulder supporting means (15).

4. Device as in any of the previous claims, wherein said counteracting structure is movable.

5. Device as in any of the previous claims, wherein said counteracting structure is implemented with one or more counterweights.

6. Device as in claim 5, wherein said counteracting structure comprises a plurality of wheels, each provided with a floor lock.

7. Device as in any of the previous claims, wherein said counteracting structure is actuator-driven.

8. Device as in any of the previous claims, comprising stopping means (16) at the end opposite the end provided with said platform for stopping movement of said table in a direction away from said platform.

9. Device as in any of the previous claims, comprising further stopping means (29) to keep said base structure in a fixed position with respect to said counteracting structure.

10. Device as in claim 9, wherein said further stopping means are a part of said counteracting structure.

11. Device as in any of the previous claims, comprising a lock to keep said table in a fixed position with respect to said base structure.

12. Device as in any of the previous claims, comprising a removable part (18) to be put on said table in front of said platform, said removable part so dimensioned as to form an angle of 90° relative to said table.

13. Device as in any of the previous claims, wherein said angle relative to said table is adjustable.

14. Method for acquiring a weight-bearing radiological image of a part of interest of a person, using a device as in any of claims 1 to 13, the method comprising :
- mounting said table (10) and base structure (11) on a scanner table of a radiological imaging device,
- positioning a person (34) with the feet on said platform (12) of said base structure and with said part of interest inside an image field of view of a radiological imaging modality scanner of said radiological imaging device,
- applying a first load to said table of said device, said first load being related to one or more features specific to said person and exerting force in a direction towards an end of said base structure provided with said platform from the opposite end of said base structure,
**characterised in** applying a second load to said base structure of said device, said second load exerting force in a direction from said one end provided with said platform towards the opposite end of said base structure, said first and said second load configured to keep said part of interest within said image field,
- acquiring said weight-bearing radiological image of said part of interest, while said person performs a movement to counter said force exerted by said first and said second load.

15. Method for acquiring as in claim 14, wherein said movement is a cyclic movement of flexion and extension.

16. Method for acquiring as in claim 14 or 15, comprising securing said table on said base structure to said radiological imaging device.

## Patentansprüche

1. Vorrichtung (1) zur Verwendung mit einem radiologischen Bildgebungsscanner unter Gewichtsbelastung, wobei die Vorrichtung Folgendes umfasst:
- eine Basisstruktur (11), die in Bezug zu einem Tisch (10) verschiebbar montiert ist, wobei die Basisstruktur an einem Ende mit einer Plattform (12) versehen ist, die einen Winkel zu dem Tisch bildet, und
- eine Gegenstruktur (20), die so angeordnet ist, dass sie auf den Tisch eine erste Last aufbringt, die von dem gegenüberliegenden Ende der Basisstruktur in Richtung des einen Endes, das mit der Plattform versehen ist, eine Kraft ausübt;
**dadurch gekennzeichnet, dass** die Gegenstruktur ferner so angeordnet ist, dass sie auf die Basisstruktur eine zweite Last aufbringt, die von dem einen Ende, das mit der Plattform versehen ist, in Richtung des gegenüberliegenden Endes der Basisstruktur eine Kraft ausübt.

2. Vorrichtung nach Anspruch 1, wobei die Basisstruktur und der Tisch aus einem röntgentransparenten Material und/oder nichtferromagnetischem Material gefertigt sind.

3. Vorrichtung nach Anspruch 1 oder 2, wobei der Tisch mit einer verstellbaren Schulterstütze (15) versehen ist.

4. Vorrichtung nach einem der vorstehenden Ansprüche, wobei die Gegenstruktur beweglich ist.

5. Vorrichtung nach einem der vorstehenden Ansprüche, wobei die Gegenstruktur mit einem oder mehreren Gegengewichten ausgestattet ist.

6. Vorrichtung nach Anspruch 5, wobei die Gegenstruktur eine Vielzahl von Rädern umfasst, die jeweils mit einer Bodenverriegelung versehen sind.

7. Vorrichtung nach einem der vorstehenden Ansprüche, wobei die Gegenstruktur durch eine Betätigungsvorrichtung Aktor angetrieben wird.

8. Vorrichtung nach einem der vorstehenden Ansprüche, umfassend Stoppmittel (16) an dem Ende, das dem Ende, das mit der Plattform versehen ist, gegenüberliegt, zum Stoppen einer Bewegung des Tisches in einer Richtung weg von der Plattform.

9. Vorrichtung nach einem der vorstehenden Ansprüche, umfassend weitere Stoppmittel (29), um die Basisstruktur in einer festen Position in Bezug auf die Gegenstruktur zu halten.

10. Vorrichtung nach Anspruch 9, wobei die weiteren Stoppmittel Teil der Gegenstruktur sind.

11. Vorrichtung nach einem der vorstehenden Ansprüche, umfassend ein Schloss, um den Tisch in einer festen Position in Bezug auf die Basisstruktur zu halten.

12. Vorrichtung nach einem der vorstehenden Ansprüche, umfassend ein abnehmbares Teil (18), das auf dem Tisch vor der Plattform platziert wird, wobei das abnehmbare Teil so dimensioniert ist, dass es einen Winkel von 90° zu dem Tisch bildet.

13. Vorrichtung nach einem der vorstehenden Ansprüche, wobei der Winkel zu dem Tisch einstellbar ist.

14. Verfahren zum Aufnehmen eines radiologischen Bildes unter Gewichtsbelastung eines relevanten Körperteils einer Person unter Verwendung einer Vorrichtung nach einem der Ansprüche 1 bis 13, wobei das Verfahren Folgendes umfasst:
- Montieren des Tisches (10) und der Basisstruktur (11) auf einem Scannertisch einer radiologischen Bildgebungsvorrichtung,
- Positionieren einer Person (34) mit den Füßen auf der Plattform (12) der Basisstruktur und mit dem relevanten Körperteil innerhalb eines Bildfelds eines radiologischen Bildgebungsscanners der radiologischen Bildgebungsvorrichtung,
- Aufbringen einer ersten Last auf die Tischplatte der Vorrichtung, wobei diese erste Last mit einem oder mehreren Merkmalen zusammenhängt, die für die Person spezifisch sind, und Ausüben einer Kraft von dem gegenüberliegenden Ende der Basisstruktur in einer Richtung eines Endes der Basisstruktur, das mit der Plattform versehen ist,
**gekennzeichnet durch** Ausüben einer zweiten Last auf die Basisstruktur der Vorrichtung, wobei die zweite Last eine Kraft in einer Richtung von dem einen Ende der Basisstruktur, das mit der Plattform versehen ist, zum gegenüberliegenden Ende der Basisstruktur ausübt, wobei die erste und die zweite Last so konfiguriert sind, dass der relevante Teil innerhalb des Bildfelds bleibt.
- Aufnehmen des radiologischen Bildes unter Gewichtsbelastung des relevanten Körperteils, während die Person eine Bewegung ausführt, um der von der ersten und der zweiten Last ausgeübten Kraft entgegenzuwirken.

15. Verfahren zum Aufnehmen nach Anspruch 14, wobei es sich bei der Bewegung um eine zyklische Beuge- und Streckbewegung handelt.

16. Verfahren zum Aufnehmen nach Anspruch 14 oder 15, umfassend Sichern des Tisches auf der Basisstruktur an der radiologischen Bildgebungsvorrichtung.

## Revendications

1. Dispositif (1) destiné à être utilisé avec un scanner de modalité d'imagerie radiologique en charge, le dispositif comprenant :
- une structure de base (11) montée de manière coulissante par rapport à une table (10), ladite structure de base étant pourvue, à une extrémité, d'une plateforme (12) formant un angle par rapport à ladite table, et
- une structure de contrepoids (20) agencée pour appliquer à ladite table une première charge exerçant une force dans une direction vers ladite extrémité pourvue de ladite plateforme depuis l'extrémité opposée de ladite structure de base ; **caractérisé en ce que** la structure de contrepoids est en outre agencée pour appliquer à ladite structure de base une seconde charge exerçant une force dans une direction depuis ladite extrémité pourvue de ladite plateforme vers l'extrémité opposée de ladite structure de base.

2. Dispositif selon la revendication 1, dans lequel ladite structure de base et ladite table sont réalisées en un matériau radiotransparent et/ou non ferromagnétique.

3. Dispositif selon la revendication 1 ou 2, dans lequel ladite table est pourvue de moyens de support d'épaule ajustables (15).

4. Dispositif selon l'une quelconque des revendications précédentes, dans lequel ladite structure de contrepoids est mobile.

5. Dispositif selon l'une quelconque des revendications précédentes, dans lequel ladite structure de contrepoids est mise en œuvre avec un ou plusieurs contrepoids.

6. Dispositif selon la revendication 5, dans lequel ladite structure de contrepoids comprend une pluralité de roues, chacune pourvue d'un verrou de sol.

7. Dispositif selon l'une quelconque des revendications précédentes, dans lequel ladite structure de contrepoids est actionnée par un actionneur.

8. Dispositif selon l'une quelconque des revendications précédentes, comprenant des moyens d'arrêt (16) à l'extrémité opposée à l'extrémité pourvue de ladite plateforme pour arrêter le mouvement de ladite table dans une direction s'éloignant de ladite plateforme.

9. Dispositif selon l'une quelconque des revendications précédentes, comprenant des moyens d'arrêt supplémentaires (29) pour maintenir ladite structure de base dans une position fixe par rapport à ladite structure de contrepoids.

10. Dispositif selon la revendication 9, dans lequel lesdits moyens d'arrêt supplémentaires font partie de ladite structure de contrepoids.

11. Dispositif selon l'une quelconque des revendications précédentes, comprenant un verrou pour maintenir ladite table dans une position fixe par rapport à ladite structure de base.

12. Dispositif selon l'une quelconque des revendications précédentes, comprenant une partie amovible (18) à placer sur ladite table devant ladite plateforme, ladite partie amovible étant dimensionnée de manière à former un angle de 90° par rapport à ladite table.

13. Dispositif selon l'une quelconque des revendications précédentes, dans lequel ledit angle par rapport à ladite table est ajustable.

14. Procédé pour acquérir une image radiologique en charge d'une partie d'intérêt d'une personne, utilisant un dispositif selon l'une quelconque des revendications 1 à 13, le procédé comprenant :
- monter ladite table (10) et ladite structure de base (11) sur une table de scanner d'un dispositif d'imagerie radiologique,
- positionner une personne (34) avec les pieds sur ladite plateforme (12) de ladite structure de base et avec ladite partie d'intérêt à l'intérieur d'un champ de vision d'une modalité d'imagerie radiologique du dispositif d'imagerie radiologique,
- appliquer une première charge à ladite table dudit dispositif, ladite première charge étant liée à une ou plusieurs caractéristiques spécifiques à ladite personne et exerçant une force dans une direction vers une extrémité de ladite structure de base pourvue de ladite plateforme depuis l'extrémité opposée de ladite structure de base, **caractérisé en ce que** l'on applique une seconde charge à ladite structure de base dudit dispositif, ladite seconde charge exerçant une force dans une direction depuis ladite extrémité pourvue de ladite plateforme vers l'extrémité opposée de ladite structure de base, ladite première et ladite seconde charge étant configurées pour maintenir ladite partie d'intérêt à l'intérieur dudit champ de vision,
- acquérir ladite image radiologique en charge de ladite partie d'intérêt, tandis que ladite personne effectue un mouvement pour contrer ladite force exercée par ladite première et ladite seconde charge.

15. Procédé d'acquisition selon la revendication 14, dans lequel ledit mouvement est un mouvement cyclique de flexion et d'extension.

16. Procédé d'acquisition selon la revendication 14 ou 15, comprenant la fixation de ladite table sur ladite structure de base au dispositif d'imagerie radiologique.
